# EUROPEAN PATENT APPLICATION

(11) **EP 4 610 419 A1**
(43) Date of publication of application: **03.09.2025**
(21) Application number: 23883021.0
(22) Date of filing: 23.10.2023
(51) Int. Cl.: D06F 34/18, D06F 33/44, A61L 2/07, D06F 39/08, D06F 34/26, D06F 34/16, D06F 103/06, D06F 103/26, D06F 103/32, D06F 105/52

(54) **METHOD FOR CONTROLLING WASHING MACHINE**

(30) Priority: 26.10.2022 KR 20220139512
(71) Applicant: LG Electronics Inc., Yeongdeungpo-gu Seoul 07336 (KR)
(72) Inventor: CHA, Seungchul, Seoul 08592 (KR); KIM, Youngho, Seoul 08592 (KR); LEE, Sangwook, Seoul 08592 (KR)
(74) Representative: Schott, Jakob Valentin
(86) International application number: PCT/KR2023/016434
(87) International publication number: WO 2024/090915

(57) **Abstract**

The present invention relates to a method for controlling a washing machine, the washing machine comprising: a tub in which washing water is stored; a drum which is rotatably provided in the tub and into which laundry is put; and a steam generator which supplies steam into the tub, wherein the method comprises a sterilization washing process to sterilize the laundry. The method for controlling a washing machine comprises: a laundry weight and laundry quality detection step of determining the laundry weight and laundry quality of the laundry; a washing step of performing a washing cycle according to the laundry weight; a sterilization step of varying the supply amount and supply time of the steam according to the laundry quality; and a rinsing step of performing a rinsing process according to the laundry weight.

## Description

### TECHNICAL FIELD

The present disclosure relates to a control method of a washing machine, and more particularly to a control method of a washing machine capable of varying a washing course in accordance with a quality of laundry loaded in the washing machine.

### BACKGROUND ART

Generally, *washing machine* is a generic term for devices configured to separate contaminants attached to laundry such as garments, bedding, etc. (also referred to as "laundry" hereinafter) using chemical decomposition actions of water and detergent and physical action such as friction, etc. between water and laundry.

Washing machines are mainly classified into stirring, vortex, and drum washing machines. Among these washing machines, the drum washing machine includes a water reservoir (or a tub) configured to store water therein, and a washing tub (or a drum) rotatably provided within the water reservoir to accommodate laundry therein.

A plurality of through holes allowing water to pass therethrough is formed at the washing tub (or the drum). Washing operation is typically divided into a washing process, a rinsing process, and a spin-drying process. Progress of such processes may be checked through a control panel (or a display) provided at an outside of the washing machine.

The washing process removes contaminants attached to laundry through frictional force between water stored in the water reservoir and laundry stored in the washing tub and chemical action of detergent contained in the water.

The rinsing process rinses laundry by supplying, to the water reservoir, water in which no detergent is dissolved. In particular, in the rinsing process, detergent absorbed in (or attached to) the laundry in the washing process is removed. In the rinsing process, fabric softener may often be supplied together with water.

The spin-drying process spin-dries laundry by rotating the washing tub at a high speed after completion of the rinsing process. Typically, the overall operation of the washing machine is completed as the spin-drying process is completed. On the other hand, in a drying washing machine, a drying process may be added after completion of the spin-drying process.

Generally, washing operation is set to be performed under different conditions in accordance with an amount of laundry loaded in the washing tub (also referred to as "laundry amount" hereinafter). For example, setting of a water supply level, a washing strength, a drainage time, a spin-drying time, etc. may be varied in accordance with a laundry amount.

Meanwhile, a washing machine including a sterilization washing process in order to obtain a sterilization effect in addition to washing performance has been developed and is commercially available. In existing washing machines, however, sterilization of laundry is performed using high-temperature steam, for sterilization washing.

However, such existing washing machines do not include a sterilization washing procedure according to a laundry quality. For this reason, laundry vulnerable to a high temperature may be damaged when high-temperature steam is used for sterilization washing.

Prior art literature (Korean Unexamined Patent Publication No. 10-2009-0127823 issued on December 14, 2009) discloses a sterilization washing method of a drum washing machine including a steam generation step (S10 to S30) of increasing an internal temperature of a drum to a first target temperature by generating steam, a steam washing step (S40) of performing steam washing by rotating the drum in a state of maintaining the internal temperature of the drum at the first target temperature or above, and a main washing step (S50 to S100) of performing main washing by rotating the drum after completion of the steam washing step.

In the sterilization washing method of the washing machine according to the prior art literature, there is a problem in that, when laundry vulnerable to a high temperature is loaded, the loaded laundry may be damaged by high-temperature steam because sterilization washing is performed through supply of the high-temperature steam, irrespective of a laundry quality.

In addition, in the sterilization washing method of the washing machine according to the prior art literature, there is a problem in that a lot of time is taken in heating for sterilization washing because steam is supplied after supply of wash water and, as such, the supplied steam should heat both the supplied wash water and the laundry.

Therefore, development of a washing procedure capable of sensing a laundry quality in accordance with loaded laundry, and varying a washing process, a rinsing process, a sterilization process, and a spin-drying process in accordance with the sensed laundry quality is required.

Meanwhile, in recent years, interest in machine learning such as artificial intelligence, deep learning, etc. is greatly increasing. Conventional machine learning was focused on statistical-based classification, regression, and clustering models. In particular, in supervised learning for classification and regression models, characteristics of training data and a machine learning model to distinguish new data based on these characteristics were predefined by humans. In contrast, deep learning allows a computer to automatically discover and differentiate features by itself.

One of the factors that accelerated the development of deep learning is open source deep learning frameworks. For example, as deep learning frameworks, there are Theano from the University of Montreal in Canada, Torch from New York University in U.S.A., Caffe from the University of California, Berkeley, TensorFlow from Google, etc. With the release of these deep learning frameworks, the importance of not only deep learning algorithms, but also learning processes, learning methods, and extraction and selection of data used for learning, has become increasingly significant for effective learning and recognition.

Additionally, research is increasing to utilize artificial intelligence and machine learning in various products and services. There is also active development to apply artificial intelligence and machine learning for sensing the amount and quality of laundry and enabling optimized washing, rinsing, sterilization, and spin-drying processes based on the sensed laundry amount and quality.

### DISCLOSURE

### TECHNICAL TASK

One technical task of the present disclosure devised to solve the problem lies in a control method of a washing machine capable of sensing the quality of laundry loaded in the washing machine.

Another technical task of the present disclosure devised to solve the problem lies in a control method of a washing machine capable of sensing the quality of laundry loaded in the washing machine and varying a washing process based on the sensed laundry quality.

Another technical task of the present disclosure devised to solve the problem lies in a control method of a washing machine capable of sensing the quality of laundry loaded in the washing machine and varying a sterilization process based on the sensed laundry quality.

Another technical task of the present disclosure devised to solve the problem lies in a control method of a washing machine capable of reducing a heating time by steam by extracting wash water from laundry before sterilization washing.

On the other hand, objects of the present disclosure are not limited to the above-described objects, and other objects of the present disclosure not yet described will be more clearly understood by those skilled in the art from the following detailed description.

### TECHNICAL SOLUTIONS

In accordance with an embodiment of the present disclosure for accomplishing the above-described objects, a control method of a washing machine equipped with a tub configured to store wash water, a drum provided in the tub to be rotatable and configured to load laundry therein, and a steam generator configured to supply steam to an interior of the tub, the washing machine including a sterilization washing procedure of performing sterilization of the laundry, may include laundry amount and quality sensing of determining a laundry amount and a laundry quality of the laundry, washing of performing a washing process in accordance with the laundry amount, sterilization of varying a supply amount and a supply time of the steam in accordance with the laundry quality, and rinsing of performing a rinsing process in accordance with the laundry amount.

The washing may further include water supply of supplying wash water to the laundry, drainage of draining the wash water used in washing after washing of the laundry, and washing-spin-drying of spin-drying the laundry to expel the wash water from the laundry, and the sterilization may be performed after the washing-spin-drying.

Additionally, the sterilization may set a sterilization temperature of the sterilization in accordance with the laundry quality sensed in the laundry amount and quality sensing.

Meanwhile, the sterilization temperature may be set in accordance with the supply amount and the supply time of the steam.

Additionally, the tub may be further equipped with a temperature sensor configured to sense a temperature inside the tub, and the sterilization temperature may be sensed by the temperature sensor.

Meanwhile, the laundry amount and quality sensing may include dry laundry amount sensing of sensing a dry laundry amount of the laundry loaded in the drum, laundry amount sensing-water supply of supplying a predetermined amount of wash water to the drum, wet laundry amount sensing of sensing a wet laundry amount of the laundry soaked with the wash water, wet laundry spin-drying of spin-drying the laundry soaked with the wash water to have the wet laundry amount, humid laundry amount sensing of sensing a humid laundry amount of the spin-dried laundry, and laundry amount/quality sensing of a laundry amount and a laundry quality based on the dry laundry amount, the wet laundry amount, and the humid laundry amount.

The laundry amount sensing-water supply may simultaneously supply the wash water and rotate the drum at a predetermined speed.

Meanwhile, the wet laundry spin-drying may further include wet laundry unbalance sensing of sensing unbalance of the laundry simultaneously with the spin-drying of the laundry.

Additionally, each of the dry laundry amount sensing, the wet laundry amount sensing, and the humid laundry amount sensing may perform the sensing thereof based on a current amount of a drive motor for rotation of the drum at a predetermined speed and an RPM of the drum during inertial rotation of the drum.

Meanwhile, the control method may further include preliminary rinsing of simultaneously performing rinsing and spin-drying of the laundry used in the washing before the sterilization.

In accordance with another embodiment of the present disclosure for accomplishing the above-described objects, a control method of a washing machine equipped with a tub configured to store wash water, a drum provided in the tub to be rotatable and configured to load laundry therein, and a steam generator configured to supply steam to an interior of the tub, the washing machine including a sterilization washing procedure of performing sterilization of the laundry, may include laundry amount and quality sensing of determining a laundry amount and a laundry quality of the laundry, washing of performing a washing process in accordance with the laundry amount, first rinsing of performing a first rinsing process and rinsing-spin-drying in accordance with the laundry amount, sterilization of varying a supply amount and a supply time of the steam in accordance with the laundry quality, and second rinsing of performing a second rinsing process in accordance with the laundry amount.

The sterilization may set a sterilization temperature of the sterilization in accordance with the laundry quality sensed in the laundry amount and quality sensing.

Meanwhile, the sterilization temperature may be set in accordance with the supply amount and the supply time of the steam.

The tub may be further equipped with a temperature sensor configured to sense a temperature inside the tub, and the sterilization temperature may be sensed by the temperature sensor.

Meanwhile, the laundry amount and quality sensing may include dry laundry amount sensing of sensing a dry laundry amount of the laundry loaded in the drum, laundry amount sensing-water supply of supplying a predetermined amount of wash water to the drum, wet laundry amount sensing of sensing a wet laundry amount of the laundry soaked with the wash water, wet laundry spin-drying of spin-drying the laundry soaked with the wash water to have the wet laundry amount, humid laundry amount sensing of sensing a humid laundry amount of the spin-dried laundry, and laundry amount/quality sensing of a laundry amount and a laundry quality based on the dry laundry amount, the wet laundry amount, and the humid laundry amount.

The laundry amount sensing-water supply may simultaneously supply the wash water and rotate the drum at a predetermined speed.

Meanwhile, the wet laundry spin-drying may further include wet laundry unbalance sensing of sensing unbalance of the laundry simultaneously with the spin-drying of the laundry.

Additionally, each of the dry laundry amount sensing, the wet laundry amount sensing, and the humid laundry amount sensing may perform the sensing thereof based on a current amount of a drive motor for rotation of the drum at a predetermined speed and an RPM of the drum during inertial rotation of the drum.

### ADVANTAGEOUS EFFECTS

In accordance with each embodiment of the present disclosure, there is an effect capable of providing a control method of a washing machine capable of sensing the quality of laundry loaded in the washing machine.

In accordance with each embodiment of the present disclosure, there is an effect capable of providing a control method of a washing machine capable of sensing the quality of laundry loaded in the washing machine and varying a washing process based on the sensed laundry quality.

In accordance with each embodiment of the present disclosure, there is an effect capable of providing a control method of a washing machine capable of sensing the quality of laundry loaded in the washing machine and varying a sterilization process based on the sensed laundry quality.

In accordance with each embodiment of the present disclosure, there is an effect capable of providing a control method of a washing machine capable of reducing a heating time by steam by extracting wash water from laundry before sterilization washing.

Effects of the present disclosure are not limited to the above-described effects. Other effects not described in the present disclosure may be clearly understood by those skilled in the art from the appended claims.

### DESCRIPTION OF DRAWINGS

FIG. 1 is a perspective view showing a washing machine according to an embodiment of the present disclosure.
FIG. 2 is a perspective view briefly showing an inner structure of the washing machine according to the embodiment of the present disclosure.
FIG. 3 is a partial sectional view of the inner structure of the washing machine according to the embodiment of the present disclosure.
FIG. 4 is a front view showing the inner structure of the washing machine according to the embodiment of the present disclosure.
FIG. 5 is a flowchart showing a sterilization washing procedure of the washing machine according to an embodiment of the present disclosure.
FIG. 6 is a flowchart showing a sterilization washing procedure according to another embodiment of the present disclosure.
FIG. 7 is a flowchart showing a laundry amount and quality sensing process in the washing machine according to an embodiment of the present disclosure.

### BEST MODE FOR DISCLOSURE

Hereinafter, a washing machine and a control method thereof according to embodiments of the present disclosure will be described in detail with reference to the accompanying drawings.

In describing the present disclosure, the name of each constituent element defined is determined taking into consideration the function of the constituent element in the present disclosure. Therefore, the defined name should not be understood as limiting the technical constituent elements of the present disclosure. Additionally, each defined name for each constituent element may be referred to by another name in the present technical field.

Therefore, the present disclosure is not limited to embodiments described below, and a person skilled in the technical field to which the present disclosure pertains would be able to make various modifications and alterations based on the following description, and the modifications and alterations should be considered as falling within the scope of the disclosure.

Hereinafter, a washing machine and a control method thereof according to embodiments of the present disclosure will be described with reference to the accompanying drawings.

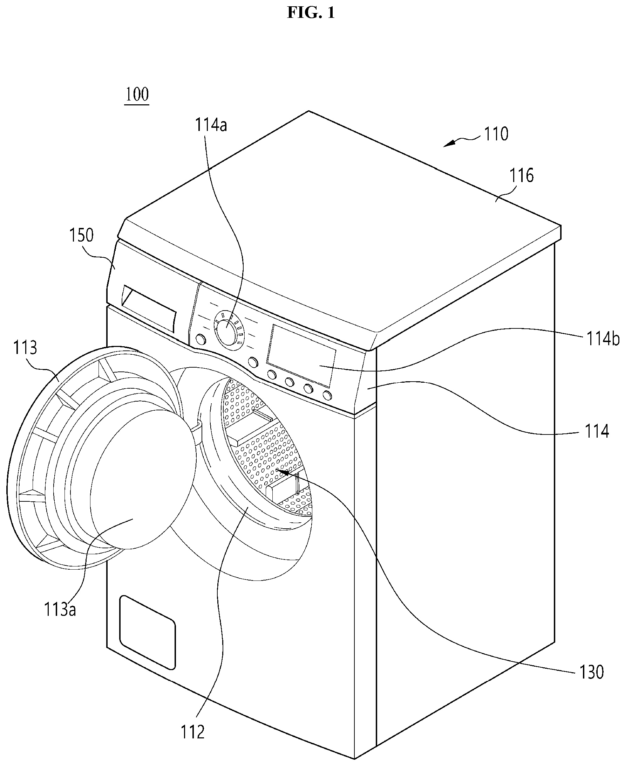

First, a washing machine according to an embodiment of the present disclosure will be described in detail with reference to FIGs. 1 to 4.

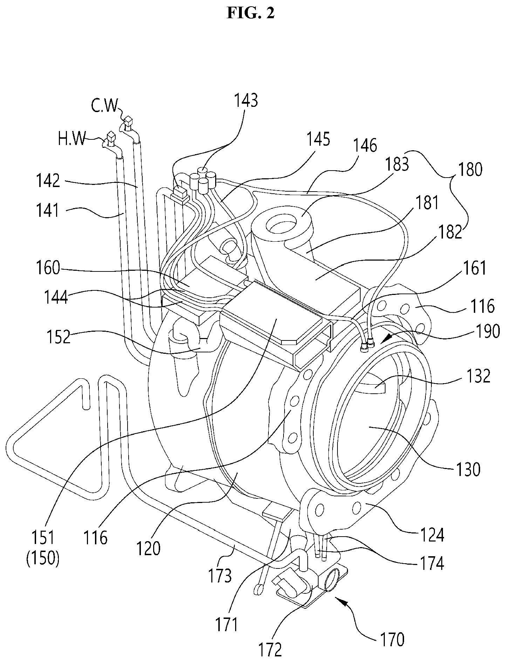

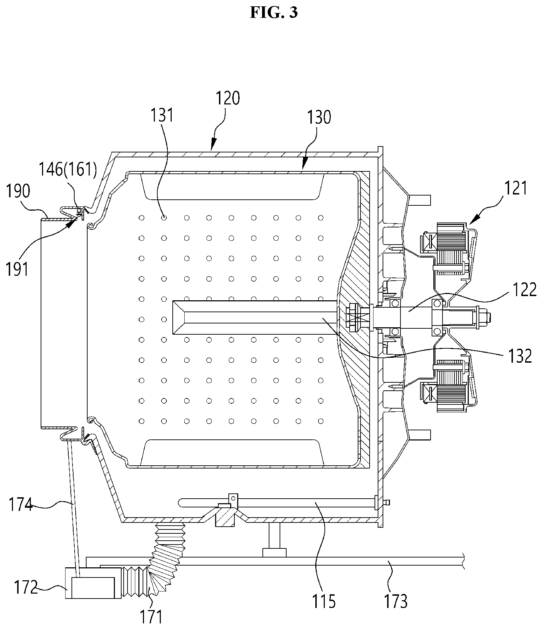

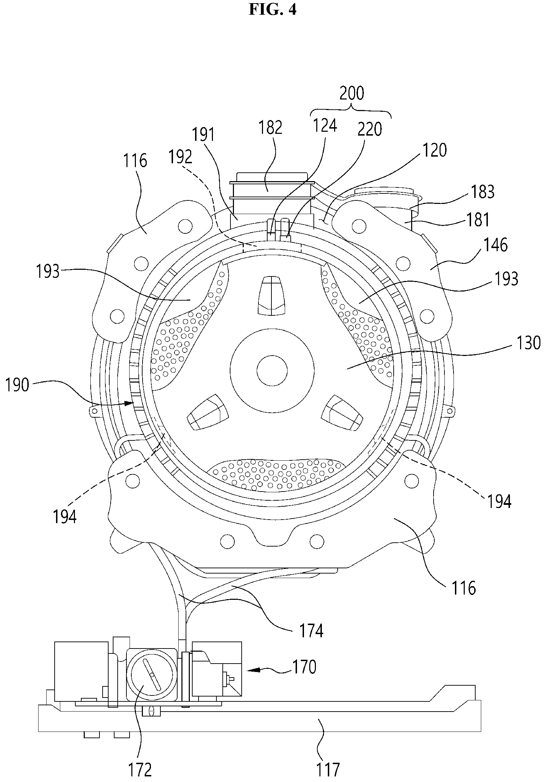

FIG. 1 is a perspective view showing the washing machine according to the embodiment of the present disclosure. FIG. 2 is a perspective view briefly showing an inner structure of the washing machine according to the embodiment of the present disclosure. FIG. 3 is a partial sectional view of the inner structure of the washing machine according to the embodiment of the present disclosure. FIG. 4 is a front view showing the inner structure of the washing machine according to the embodiment of the present disclosure.

As shown in the drawings, the washing machine 100 according to the embodiment of the present disclosure may include a cabinet 110 configured to form an outer shape of the washing machine 100, a tub 120 provided within the cabinet 110 and configured to store wash water, a drum 130 rotatably provided within the tub 120, a water supplier140 configured to supply wash water to the tub 120, a detergent dispenser 150 configured to mix water supplied from the water supplier 140 with detergent and to supply the mixture to the tub 120, a steam generator 160 configured to heat water supplied from the water supplier 140 and to supply steam to the tub 120, a drain unit 170 configured to discharge wash water used to complete washing, and a drying device 180 configured to heat air in the tub 120 and to supply/circulate the heated air.

Meanwhile, the washing machine of the present disclosure is provided with a gasket 190 between the cabinet 110 and the tub 120. The gasket 190 prevents water stored in the tub 120 from leaking between the tub 120 and the cabinet 110.

In this case, a water supply nozzle 192 configured to supply water supplied from water supply sources HW and CW not only to an interior of the drum 130, but also to an inner surface of the gasket 190, may be provided at an upper portion of the gasket 190. Circulation nozzles 194 configured to again supply the wash water supplied to the tub 120 in a circulation manner after being circulated by the drain unit 170 may be provided at opposite sides of the gasket 190.

The cabinet 110 forms the exterior of the washing machine 100 and may include side cabinets 115 configured to form side surfaces of the washing machine 100, a base 117 configured to form a bottom surface of the washing machine 100, a front cabinet 111 formed with a laundry access hole 112 configured to allow loading and unloading of laundry therethrough and coupled to front surfaces of the side cabinets 115, a top cabinet 116 provided at upper sides of the side cabinets 115, and a door 113 pivotably coupled to the front cabinet 111 to open and close the laundry access hole 112.

Meanwhile, a control panel 114 is provided at an upper portion of the front cabinet 111. The control panel 114 may include a course selector 114a configured to receive a course selection input from the user, and a display 114b configured to receive various control commands from the user and to display an operation state of the washing machine 100.

In addition, the door 113 may be equipped with a glass 113a configured to enable laundry in the drum 130 to be observed from an outside of the washing machine 100. The glass 113a may be formed to have a convex shape. When the door 113 is closed, an inner end of the glass 113a may protrude to an inside of the drum 130.

The tub 120 may be provided inside the cabinet 110 in a suspended manner. A drive motor 121 and a rotating shaft 122 configured to rotate the drum 130 may be provided at the rear of the tub 120. Here, the drum 130 may be divided into a direct driving type and an indirect driving type in accordance with a method of transmitting driving force provided from the drive motor 121 to the drum 130.

Here, in the direct driving type, the rotating shaft of the drive motor 121 is directly coupled to the drum 130, and the rotating shaft of the drive motor 121 and the center of the drum 130 are aligned with each other along the same line. The washing machine 100 according to the present embodiment employs this direct drive type and, as such, the drum 130 is rotated by the drive motor 121 provided at the rear of the tub 120. However, the present disclosure is not necessarily limited to the above-described configuration, and the indirect drive type, which will be described later, may also be possible.

On the other hand, in the indirect driving type, the drum 130 is rotated using a power transmission means such as a belt, a pulley or the like configured to transmit driving force provided from the drive motor 121. In this case, the rotating shaft of the drive motor 121 and the center of the drum 130 do not necessarily need to be aligned with each other along the same line.

The tub 120 as described above may be suspended from the cabinet 110 by a spring (not shown) so that vibrations generated during rotation of the drum 130 may be dampened. A damper (not shown) configured to support the tub 120 from below may also be provided.

In addition, a heater 123 configured to heat water stored in the tub 120 and a temperature sensor (not shown) configured to sense an internal temperature of the tub 120 may be further provided within the tub 120. Meanwhile, a weight 116 may be provided at a front portion of the tub 120 to increase the weight of the tub 120 itself and, as such, to raise a vibration limit of the tub 120.

The drum 130 is connected to the rotating shaft inside the tub and is configured to receive rotational force of the drive motor 121 so as to be rotatable. A plurality of through holes 131 is formed at an inner circumferential surface of the drum 130 in order to enable wash water stored in the tub 120 to flow into the drum 130. In addition, one or more lifters 132 may be provided at an inner surface of the drum 130 along the inner circumferential surface of the drum 130 in order to enable tumbling of laundry loaded in the drum 130 by rotation of the drum 130.

Meanwhile, the tub 120 and the drum 130 may be disposed in a horizontal state, but alternatively, may be disposed to have a predetermined inclination so that rear portions of the tub 120 and the drum 130 are lower than front portions of the tub 120 and the drum 130.

The water supplier 140 may be equipped with a plurality of water supply valves 143 and a plurality of water supply hoses 144, 145 and 146 configured to supply watersupplied from the external water supply sources HW and CW to the tub 120, the detergent dispenser 150, the steam generator 160, etc.

Here, the water supplier 140 is connected to the hot water supply source HW configured to supply hot water and a cold water supply source CW configured to supply cold water through a hot water hose 141 and a cold water hose 142, respectively, and water introduced through the hot water hose 141 and the cold water hose 142 may be supplied to the detergent dispenser 150, the steam generator 160, and/or the water supply nozzle 192 through appropriate control of the water supply valve 143.

The water supplier 140 described above may include a dispenser water supply hose 144 configured to guide water to the detergent dispenser 150, a steam water supply hose 145 configured to guide water to the steam generator 160, and a front water supply hose 146 configured to guide water to the water supply nozzle 192.

The detergent dispenser 150 temporarily stores additives such as detergent for preliminary washing or main washing, fabric softener, bleach or the like, and may mix the additives with the wash water supplied according to the water supply of the water supplier 140 and may supply the mixture to the tub 120.

Meanwhile, the detergent dispenser 150 may be provided with a plurality of partitioned storage spaces (not shown) configured to separately store additives while preventing intermingling of the additives. Water may be supplied to the storage spaces in an individual manner.

The detergent dispenser 150 described above includes a detergent dispenser housing 151 formed to have a shape recessed into the interior of the cabinet 110, and the detergent dispenser 150 may be configured in a drawer form so that the detergent dispenser 150 may be withdrawn from the detergent dispenser housing 151 towards the front of the cabinet 110.

In this case, the detergent dispenser housing 151 communicates with the tub 120 through a water supply bellows 152. Accordingly, water supplied from the water supplier 140 may be mixed with additives while passing through the detergent dispenser 150, and may then move to the tub 120 along the water supply bellows 152 connected to the detergent dispenser housing 151.

The steam generator 160 is a device configured to heat the water supplied from the water supplier 140 to produce steam. The steam generator 160 may supply the generated steam from the front of the drum 130 to perform functions such as sterilization, wrinkle removal, etc. of the laundry within the drum 130.

The drain unit 170 is provided under the tub 120 to drain wash water used to complete washing in the tub 120. The drain unit 170 may include a drain bellows 171 connected to a lower portion of the tub 120, a drain pump 172 configured to pump wash water drained through the drain bellows 171, a drain hose 173 configured to drain wash water moved by the drain pump 172, and a circulation hose 174 configured to circulate the wash water moved by the drain pump 172 to the tub 120.

Here, the drain hose 173 may extend to the outside of the washing machine 100 under the condition that a trap is formed at a portion of the drain hose 173 disposed at the outside of the washing machine 100. The circulation hose 174 may be branched into at least two portions and may be connected to the circulation nozzles 194 formed at opposite sides of the gasket 190.

Accordingly, when the drain pump 172 operates, wash water in the tub 120 may be drained through the drain hose 173 or wash water in the tub 120 may be again supplied to the tub 120 through the circulation nozzles 194. For this function, a separate 3-way valve (not shown) configured to set a flow path for selective movement of water moved by the drain pump 172 to the drain hose 173 or the circulation hose 174 may be provided at the drain pump 172.

Although the drain pump 172 has been described as having both the function of a drain pump and the function of a circulation pump in the present embodiment, a pump for drainage and a pump for circulation may be separately provided, differently from the above configuration.

The drying device 180 heats and circulates air in the tub 120, and guides flow of air such that the circulating heated air is again guided to the interior of the tub 120. The drying device 180 may include an intake duct 181 configured to suck in air from the tub 120, and a supply duct 182 configured to guide the moved air into the tub 120.

Meanwhile, a blower fan 183 configured to create pressure for moving air is provided between the intake duct 181 and the supply duct 182. The intake duct 181 connects a rear outer circumferential surface of the tub 120 to the blower fan 183, and the supply duct 182 connects the blower fan 183 to the gasket 190. In addition, a heater (not shown) may also be provided within the supply duct 182 to heat air moving through the supply duct 182.

The gasket 190 is coupled to the front cabinet 111 at one side thereof and is coupled to a circumference of an opened front portion of the tub 120 at the other side thereof. The gasket 190 described above may be formed in a bellows shape so that the gasket 190 elastically folds in response to vibrations of the tub 120, thereby dampening the vibrations. In this case, the gasket 190 may be made of a deformable or flexible material having a certain degree of elasticity and may be formed using natural rubber or synthetic resin.

Meanwhile, as described above, a duct connection portion 191, to which the supply duct 180 of the drying device 180 is connected, is formed at an upper rear portion of the gasket 190. In addition, a water supply nozzle 192 is disposed at an upper front side of the gasket 190, and a plurality of circulation nozzles 194 configured to spray water into the drum 130 may be formed at opposite sides of a lower portion of the inner circumferential surface of the gasket 190. The circulation hose 174 extending from the drain unit 170 may be connected to each circulation nozzle 194.

In addition, anti-jamming protrusions 193 are formed at opposite sides of an upper portion of the inner circumferential surface of the gasket 190 to protrude toward the center of the gasket 190 in a symmetrical manner. The anti-jamming protrusions 193 are configured to not only prevent laundry from dislodging from the drum 130 due to rotation of the drum 130 and getting stuck between the gasket 190 and the cabinet 110, in particular, the front cabinet 111, but also to prevent the laundry from spilling out when the door 113 is opened after washing is completed.

Preferably, the anti-jamming protrusions 193 may be formed to protrude from the inner circumferential surface of the gasket 190 toward the laundry access hole. In addition, the anti-jamming protrusions 193 may be formed at multiple positions and, in particular, may be formed at positions symmetrical with respect to a vertical center of the gasket 190, respectively.

The plurality of circulation nozzles 194 is effective in uniformly moistening the laundry loaded in the drum 130 by spraying wash water from the lower portion of the gasket 190 toward the rear of the drum 130.

Hereinafter, operation of the washing machine according to the embodiment of the present disclosure will be described in detail through examples. Each of the elements described below should be understood with reference to the above description and drawings.

Meanwhile, the control method of the washing machine according to the embodiment of the present disclosure relates to a sterilization washing procedure according to a laundry quality. Accordingly, description of a general washing procedure will be omitted, and a sterilization washing process, which is a subject matter of the present disclosure, will be described in detail.

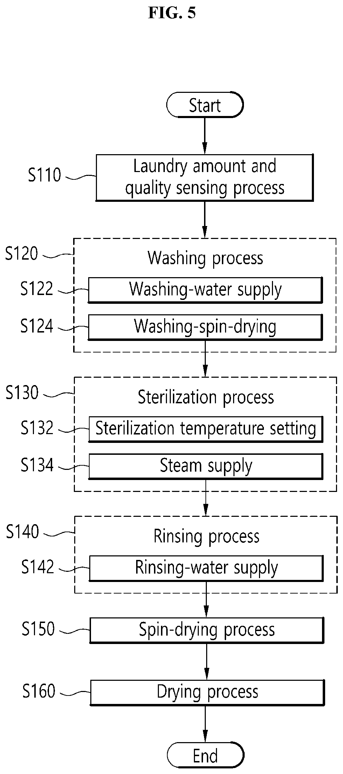

FIG. 5 is a flowchart showing a sterilization washing procedure of the washing machine according to an embodiment of the present disclosure.

First, the sterilization washing procedure according to the embodiment of the present disclosure will be described with reference to FIG. 5.

As shown in the drawing, the sterilization washing procedure according to the embodiment of the present disclosure may include a laundry amount and quality sensing process S110, a washing process S120, a sterilization process S130, a rinsing process S140, and a spin-drying process S150. In addition, a separate drying process S160 may be further performed after completion of the spin-drying process S150.

As the washing procedure begins, the laundry amount and quality sensing process S110 is carried out to sense the amount of laundry loaded in the drum 130. The laundry amount and quality sensing process S110 will be described in detail with reference to separate drawings after the sterilization washing procedure is described.

The washing process S120 is a process for removing contaminants from laundry by soaking the laundry with water mixed with wash detergent and then rotating a drum. The washing process S120 may include a washing-water supply step S122, a laundry treatment step (not shown), a washing-drainage step (not shown), a laundry distribution step (not shown), and a washing-spin-drying step S124.

Here, the washing-water supply step S122 is a step of supplying water from an external water supply source to the interior of the tub 120 and soaking laundry with water mixed with detergent. The amount of wash water supplied in the washing-water supply step S122 may be varied based on the amount and quality of laundry determined in the laundry amount and quality sensing process S110.

Meanwhile, the laundry treatment step is a step of removing contaminants from laundry by rotating the drum 130 at various speeds and/or directions to apply mechanical force such as bending force, frictional force, impact force, or the like to the laundry. The washing-drainage step is a step of discharging wash water from the tub 120 to the outside of the cabinet 110 after washing is completed. The laundry distribution step is a step of preventing generation of noise and vibrations caused by eccentricity of laundry during the washing-spin-drying step S124 by repeating acceleration and deceleration of the drum 130 to distribute the laundry.

In addition, the washing-spin-drying step S124 is a step of rotating the drum 130 at a high speed to allow the water soaked in the laundry to be expelled. During the washing-spin-drying step S124, the high-speed rotation of the drum 130 causes the laundry to rotate in a state of adhering to the inner circumferential surface of the drum 130 and, as such, water is expelled from the laundry by centrifugal force.

In the washing-spin-drying step S124, the laundry from which wash water has been expelled may be heated to a sterilization temperature by steam supplied in the sterilization process S130 which will be described later. In this case, the amount of wash water remaining in the laundry after expulsion of wash water in the washing-spin-drying step S124 is relatively less than the amount of wash water absorbed before the expulsion of wash water. Accordingly, the time required to heat the laundry to the sterilization temperature with the steam supplied in the sterilization process S130 is reduced, and the amount of steam supplied for heating to the sterilization temperature in the sterilization process S130 may also be decreased.

The sterilization process S130 is configured to remove contaminants and bacteria remaining in the laundry washed and spin-dried through the washing process by applying high-temperature steam to the laundry. In the sterilization process S130, the sterilization temperature may be set in accordance with the laundry amount and quality determined in the laundry amount and quality sensing process S110. Additionally, the drum 130 may be reciprocally rotated at a predetermined speed and at a predetermined interval to enhance contact between the steam and the laundry during supply of steam.

Meanwhile, a variety of laundry items having a wide variety of qualities may be put into the washing machine. For example, the laundry may be made of a variety of materials such as cotton, acrylic, polyester, wool, etc., and the materials may be categorized in detail based on water absorption capacity, blending ratio, etc.

Additionally, the materials of these laundry items may undergo deformation due to temperature changes depending on the washing or sterilization temperature. When laundry is described with reference to, for example, a change thereof according to temperature, laundry items may be classified into laundry made of a cotton material, which is relatively resistant to high temperature (referred to as a "first quality" hereinafter) and laundry made of a polyester material, which is temperature-sensitive and may be damaged when exposed to high-temperature steam (referred to as a "second quality" hereinafter).

Here, although laundry qualities have been classified into the first quality and the second quality, the laundry qualities may also be classified into more categories based on appropriate wash water temperatures and appropriate steam temperatures according to qualities of materials (for example, cotton, acrylic, polyester, wool, etc.). This does not limit the classification of laundry qualities.

Meanwhile, laundry of the first quality is sensitive to washing and sterilization temperatures, and may be damaged when relatively high-temperature wash water or steam is supplied. In contrast, laundry of the second quality is less sensitive to a relatively high temperature and, as such, may exhibit reduced risk of damage even from high-temperature wash water or steam.

Therefore, in the sterilization process S130, the steam supplied to heat the laundry in the tub 120 to a sterilization temperature should be adjusted according to the quality of the laundry put into the washing machine. That is, the sterilization temperature in the sterilization process S130 may be set differently based on laundry qualities sensed in the laundry amount and quality sensing process S110, and the supply time and amount of steam may be varied accordingly.

That is, in the sterilization process S130, when the laundry quality sensed in the laundry amount and quality sensing process S110 is classified as the first quality or the second quality, a sterilization temperature setting step S132 may be performed to set a steam temperature based on the sensed laundry quality, and a steam supply step S134 may be performed to supply steam for a predetermined time in order to heat the interior of the tub 120 to the set sterilization temperature.

In detail, when the laundry quality sensed in the laundry amount and quality sensing process S110 is the first quality, no deformation or damage occurs due to relatively high-temperature wash water and steam. In this case, accordingly, the sterilization temperature inside the tub 120 in the sterilization process S130 may be set to 60°C or more. On the other hand, when the laundry quality sensed in the laundry amount and quality sensing process S110 is the second quality, deformation and damage may occur due to relatively high-temperature wash water and steam. In this case, accordingly, the sterilization temperature inside the tub 120 in the sterilization process S130 may be set to 60°C or less. Preferably, the sterilization temperature for the first quality may be set between 60°C and 80°C, whereas the sterilization temperature for the second quality may be set between 40°C and 60°C.

Meanwhile, in the steam supply step S134, the drum 130 is rotated simultaneously with the supply of steam to ensure uniform contact between the loaded laundry and the steam. In this step, the rotating drum 130 may be repeatedly rotated at a predetermined speed/a predetermined interval in one direction and then in the opposite direction.

Accordingly, as the steam and the laundry uniformly contact each other through rotation of the drum 130, the temperature of the laundry may increase uniformly, and the temperature inside the tub 120 may be heated to the sterilization temperature relatively rapidly, as compared to a state in which the drum 130 is stopped.

Additionally, in the steam supply step S134, a temperature sensor may sense whether the temperature inside the tub 120 has reached the sterilization temperature, and when the temperature inside the tub 120 reaches the sterilization temperature, supply of steam and rotation of the drum 130 may be stopped.

The rinsing process S140 is a process for soaking the laundry in wash water, then rotating the drum 130 to remove residual wash detergent from the laundry, and, at the same time, cooling the laundry heated in the sterilization process S130. The rinsing process S140 may be performed at least one or more times, and in a final rinsing process S140, washing aid such as fabric softener or the like may be added, mixed with the wash water, and act on the laundry.

In this case, the rinsing process S140 is carried out in an order of a rinsing-water supply step S142, a laundry treatment step, a rinsing-drainage step, a laundry distribution step, and a rinsing-spin-drying step. The rinsing-water supply step S142, the laundry treatment step, the rinsing-drainage step, the laundry distribution step, and the rinsing-spin-drying step in the rinsing process S140 are identical to those in the washing process S120 and, as such, detailed description thereof will be omitted.

The spin-drying process S150 is a process for rotating the drum 130 at a high speed to ultimately remove moisture from the laundry. The spin-drying process S150 is carried out in an order of drainage of draining water in the tub 120 to the outside of the cabinet 110, laundry distribution of repeating acceleration and deceleration of the drum 130 to distribute the laundry, and final spin-drying of rotating the drum 130 at a high speed to remove moisture from the laundry. In the final spin-drying, the drum 130 may rotate at the maximum rotation speed higher than the maximum rotation speed thereof in the washing-spin-drying step S124 or the rinsing-spin-drying step.

The drying process S160 is configured to dry the laundry in the drum 130 by heating and circulating air in the drum 130 through operation of the drying device 180. The drying process may dry the laundry in the drum 130 by heating air in the drum 130 and supplying the heated air in a circulation manner through operation of the heater provided in the supply duct 182 of the drying device 180 and operation of the blower fan 183. In this case, it may be possible to enhance a laundry drying effect by rotating the drum 130 along with supply/circulation of heated air.

Hereinafter, operation of a washing machine according to another embodiment of the present disclosure will be described in detail through examples. Elements, which will be described hereinafter, should be understood with reference to the above description and drawings.

Meanwhile, a control method of the washing machine according to the other embodiment of the present disclosure relates to a sterilization washing procedure based on a laundry quality. Accordingly, description of a general washing procedure will be omitted, and a sterilization washing procedure, which is a subject matter of the present disclosure, will be described in detail.

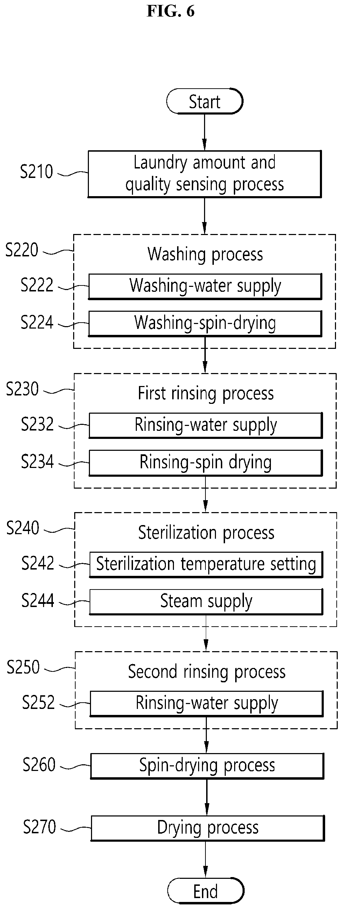

FIG. 6 is a flowchart showing a sterilization washing procedure according to the other embodiment of the present disclosure.

First, the sterilization washing procedure according to the other embodiment of the present disclosure will be described with reference to FIG. 6.

As shown in the drawing, the sterilization washing procedure according to the other embodiment of the present disclosure may include a laundry amount and quality sensing process S210, a washing process S220, a first rinsing process S230, a sterilization process S240, a second rinsing process S250, and a spin-drying process S260. In addition, a separate drying process S270 may be further performed after completion of the spin-drying process S260.

As the washing procedure begins, the laundry amount and quality sensing process S210 is carried out to sense the amount of laundry loaded in the drum 130. The laundry amount and quality sensing processes S110 and S210 according to respective embodiments of the present disclosure will be described in detail with reference to separate drawings after the sterilization washing procedure is described in association with the laundry amount and quality sensing process S210.

The washing process S220 is a process for removing contaminants from laundry by soaking the laundry with water mixed with wash detergent and then rotating a drum. The washing process S220 may include a washing-wash water supply step S222, a laundry treatment step (not shown), a washing-drainage step (not shown), a laundry distribution step (not shown), and a washing-spin-drying step S224.

Here, the washing-wash water supply step S222 is a step of supplying water from an external water supply source to the interior of a tub 120 and soaking laundry with water mixed with detergent. The amount of wash water supplied in the washing-wash water supply step S222 may be varied based on the amount and quality of laundry determined in the laundry amount and quality sensing process S210.

Meanwhile, the laundry treatment step is a step of removing contaminants from laundry by rotating the drum 130 at various speeds and/or directions to apply mechanical force such as bending force, frictional force, impact force, or the like to the laundry. The washing-drainage step is a step of discharging wash water from the tub 120 to the outside of the cabinet 110 after washing is completed. The laundry distribution step is a step of preventing generation of noise and vibrations caused by eccentricity of laundry during the washing-spin-drying step S224 by repeating acceleration and deceleration of the drum 130 to distribute the laundry.

In addition, the washing-spin-drying step S224 is a step of rotating the drum 130 at a high speed to allow the water soaked in the laundry to be expelled. During the washing-spin-drying step S224, the high-speed rotation of the drum 130 causes the laundry to rotate in a state of adhering to the inner circumferential surface of the drum 130 and, as such, water is expelled from the laundry by centrifugal force.

The first rinsing process S230 is a process for soaking the laundry in wash water and then rotating the drum 130 to remove residual laundry detergent from the laundry. In this case, the rinsing process S230 may be carried out in an order of a rinsing-water supply step S232, a laundry treatment step, a rinsing-drainage step, a laundry distribution step, and a rinsing-spin-drying step S234. The rinsing-water supply step S232, the laundry treatment step, the drainage step, and the laundry distribution step in the rinsing process S230 are identical to those in the washing process S220 and, as such, detailed description thereof will be omitted.

In the rinsing-spin-drying step S234, the laundry from which wash water has been expelled may be heated to a sterilization temperature by steam supplied in the sterilization process S240 which will be described later. In this case, the amount of wash water remaining in the laundry after expulsion of wash water in the washing-spin-drying step S234 is relatively less than the amount of wash water absorbed before the expulsion of wash water. Accordingly, the time required to heat the laundry to the sterilization temperature with the steam supplied in the sterilization process S240 is reduced, and the amount of steam supplied for heating to the sterilization temperature in the sterilization process S240 may also be decreased.

The sterilization process S240 is configured to remove contaminants and bacteria remaining in the laundry washed and spin-dried through the washing process by applying high-temperature steam to the laundry. In the sterilization process S240, the sterilization temperature may be set in accordance with the laundry amount and quality determined in the laundry amount and quality sensing process S210. Additionally, the drum 130 may be reciprocally rotated at a predetermined speed and at a predetermined interval to enhance contact between the steam and the laundry during supply of steam.

Meanwhile, a variety of laundry items having a wide variety of qualities may be put into the washing machine. For example, the laundry may be made of a variety of materials such as cotton, acrylic, polyester, wool, etc., and the materials may be categorized in detail based on water absorption capacity, blending ratio, etc.

Additionally, the materials of these laundry items may undergo deformation due to temperature changes depending on the washing or sterilization temperature. When laundry is described with reference to, for example, a change thereof according to temperature, laundry items may be classified into laundry made of a cotton material, which is relatively resistant to a high temperature (referred to as a "first quality" hereinafter) and laundry made of a polyester material, which is temperature-sensitive and may be damaged when exposed to high-temperature steam (referred to as a "second quality" hereinafter).

Here, although laundry qualities have been classified into the first quality and the second quality, the laundry qualities may also be classified into more categories based on appropriate wash water temperatures and appropriate steam temperatures according to qualities of materials (for example, cotton, acrylic, polyester, wool, etc.). This does not limit the classification of laundry qualities.

Meanwhile, laundry of the first quality is sensitive to washing and sterilization temperatures, and may be damaged when relatively high-temperature wash water or steam is supplied. In contrast, laundry of the second quality is less sensitive to relatively high temperatures and, as such, may exhibit reduced risk of damage even from high-temperature wash water or steam.

Therefore, in the sterilization process S240, the steam supplied to heat the laundry in the tub 120 to a sterilization temperature should be adjusted according to the quality of the laundry put into the washing machine. That is, the sterilization temperature in the sterilization process S240 may be set differently based on laundry qualities sensed in the laundry amount and quality sensing process S210, and the supply time and amount of steam may be varied accordingly.

That is, in the sterilization process S240, when the laundry quality sensed in the laundry amount and quality sensing process S210 is classified as the first quality or the second quality, a sterilization temperature setting step S242 may be performed to set a steam temperature based on the sensed laundry quality, and a steam supply step S244 may be performed to supply steam for a predetermined time in order to heat the interior of the tub 120 to the set sterilization temperature.

In detail, when the laundry quality sensed in the laundry amount and quality sensing process S210 is the first quality, no deformation or damage occurs due to relatively high-temperature wash water and steam. In this case, accordingly, the sterilization temperature inside the tub 120 in the sterilization process S240 may be set to 60°C or more. On the other hand, when the laundry quality sensed in the laundry amount and quality sensing process S210 is the second quality, deformation and damage may occur due to relatively high-temperature wash water and steam. In this case, accordingly, the sterilization temperature inside the tub 120 in the sterilization process S240 may be set to 60°C or less. Preferably, the sterilization temperature for the first quality may be set between 60°C and 80°C, whereas the sterilization temperature for the second quality may be set between 40°C and 60°C.

Meanwhile, in the steam supply step S244, the drum 130 is rotated simultaneously with the supply of steam to ensure uniform contact between the loaded laundry and the steam. In this step, the rotating drum 130 may be repeatedly rotated at a predetermined speed/a predetermined interval in one direction and then in the opposite direction.

Accordingly, as the steam and the laundry uniformly contact each other through rotation of the drum 130, the temperature of the laundry may increase uniformly, and the temperature inside the tub 120 may be heated to the sterilization temperature relatively rapidly, as compared to a state in which the drum 130 is stopped.

Additionally, in the steam supply step S244, a temperature sensor may sense whether the temperature inside the tub 120 has been heated to the sterilization temperature, and when the temperature inside the tub 120 reaches the sterilization temperature, supply of steam and rotation of the drum 130 may be stopped.

The second rinsing process S250 is a process for soaking the laundry in wash water, then rotating the drum 130 to remove residual wash detergent from the laundry, and, at the same time, cooling the laundry heated in the sterilization process S240. The rinsing process S250 may be performed at least one or more times, and in a final rinsing process S250, washing aid such as fabric softener or the like may be added, mixed with the wash water, and act on the laundry.

In this case, the second rinsing process S250 is carried out in an order of a rinsing-water supply step S252, a laundry treatment step, a rinsing-drainage step, a laundry distribution step, and a rinsing-spin-drying step. The rinsing-water supply step S252, the laundry treatment step, the rinsing-drainage step, the laundry distribution step, and the rinsing-spin-drying step in the rinsing process S250 are identical to those in the washing process S220 and, as such, detailed description thereof will be omitted.

The spin-drying process S260 is a process for rotating the drum 130 at a high speed to ultimately remove moisture from the laundry. The spin-drying process S260 is carried out in an order of drainage of draining water in the tub 120 to the outside of the cabinet 110, laundry distribution of repeating acceleration and deceleration of the drum 130 to distribute the laundry, and final spin-drying of rotating the drum 130 at a high speed to remove moisture from the laundry. In the final spin-drying, the drum 130 may rotate at the maximum rotation speed higher than the maximum rotation speed thereof in the washing-spin-drying step S224 or the rinsing-spin-drying step S234.

The drying process S270 is configured to dry the laundry in the drum 130 by heating and circulating air in the drum 130 through operation of a drying device 180. The drying process may dry the laundry in the drum 130 by heating air in the drum 130 and supplying the heated air in a circulation manner through operation of a heater provided in a supply duct 182 of the drying device 180 and operation of a blower fan 183. In this case, it may be possible to enhance a laundry drying effect by rotating the drum 130 along with supply/circulation of heated air.

Meanwhile, in each of the washing processes S120 and S220, the rinsing processes S140, S230 and S250, and the spin-drying processes S150 and S260, a wash water supply amount, a rotation speed of the drum 130, an operation time/interval of the drive motor 121, and an operation time of the drying device may be set based on the laundry amount calculated in the laundry amount and quality sensing process S110 or S210.

Additionally, in each of the sterilization processes S130 and S240, a supply time and amount of steam during the sterilization process may be set based on the laundry quality determined in the laundry amount and quality sensing process S110 or S210.

Therefore, sensing of a correct laundry amount and a correct laundry quality in the laundry amount and quality sensing processes S110 and S210 is very important.

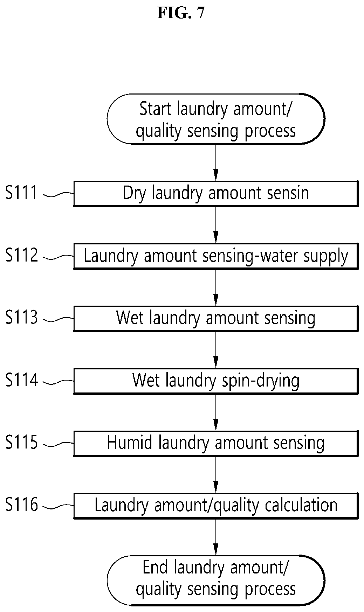

Hereinafter, laundry amount and quality sensing according to an embodiment of the present disclosure will be described in detail with reference to FIG. 7.

FIG. 7 is a flowchart showing a laundry amount and quality sensing process in the washing machine according to an embodiment of the present disclosure.

Meanwhile, the laundry amount and quality sensing processes S110 and S210 according to the present disclosure may be equally applied to each of the above-described embodiments. Accordingly, the laundry amount and quality sensing processes S110 and S210 will be described as a single procedure without being individually described. However, this does not limit procedures to which the laundry amount and quality sensing processes S110 and S210 are applicable, and the laundry amount and quality sensing processes S110 and S210 may be implemented through application thereof to other procedures in which the laundry amount and quality sensing processes S110 and S210 are necessary.

As shown in FIG. 7, each of the laundry amount quality sensing processes S110 and S210 according to the present disclosure may mainly include a dry laundry amount sensing step S111, a laundry amount sensing-water supply step S112, a wet laundry amount sensing step S113, a wet laundry spin-drying step S114, a humid laundry amount sensing step S115, and a laundry amount/quality calculation step S116.

The dry laundry amount sensing step S111 includes detecting an amount of current consumed by the drive motor 121 while rotating the drum 130, in which laundry is loaded, at a predetermined speed for a predetermined time by the drive motor 121, and detecting an RPM of the drum 130 through a Hall sensor (not shown) equipped in the drive motor 121 when the drum 130 rotates by inertia in accordance with the weight of the laundry after cutting off power supplied to the drive motor 121.

Here, the dry amount of the laundry put into the drum 130 may be calculated by comparing the detected current amount of the drive motor 121 and the detected inertial RPM of the drum 130 with a predetermined dry laundry table (not shown).

The laundry amount sensing-water supply step S112 is configured to supply a predetermined amount of wash water to the laundry loaded in the drum 130 and to measure a wet amount of the laundry in a soaked state. The wet laundry amount sensing in the laundry amount sensing-water supply step S112 may be achieved by detecting an amount of current required for driving of the drum 130 and an inertial RPM of the drum 130, and comparing the detected current amount of the drive motor 121 and the detected inertial RPM of the drum 130 with a wet laundry table (not shown), similarly to the dry laundry amount sensing step S111.

The laundry amount sensing-water supply step S112 is configured to determine a moisture content of the laundry in order to determine a quality of the laundry. That is, laundry quality may be defined based on several factors such as the material of the laundry, the softness level (for example, soft laundry/hard laundry), the ability of the laundry to absorb water (water content), the ability of the laundry to hold water (moisture content), the volume difference between laundry in a dry state and laundry in a wet state, etc. That is, laundry items having general qualities may have different water absorption abilities to absorb water in accordance with different materials. Accordingly, the amount and the quality of laundry may be sensed in accordance with the water absorption level of the laundry.

For determination of an accurate laundry quality, accordingly, a water content of laundry may be determined in order to accurately determine characteristics of the quality of the laundry when only a dry amount of the laundry is used. That is, in the case of calculating a dry laundry amount using the current amount of the driving motor 121 and the inertial RPM of the drum 130 in the dry laundry amount sensing step S111, it is difficult to determine an accurate laundry quality only through simple determination of the dry laundry amount. Accordingly, in addition to the dry laundry amount calculated in the dry laundry amount sensing step S111, a laundry amount sensing-water supply step S112 of supplying a predetermined amount of wash water may be performed to determine a wet amount of laundry soaked with wash water. In this case, the amount of supplied wash water may range from about 1.5 to 2.5L, and preferably, 2L of wash water may be supplied.

Additionally, in the laundry amount sensing-water supply step S112, the drum 130 may be rotated at a predetermined speed to ensure that the laundry more effectively absorbs the supplied wash water. Moreover, the wash water supplied to the drum 130 may be directly supplied to the interior of the drum 130 through the water supply nozzle 192 or the circulation nozzles 194 disposed at the gasket 190.

The wet laundry amount sensing step S113 is configured to sense a wet laundry amount of the laundry loaded in the drum 130 and soaked with wash water. The wet laundry amount sensing step S113 may sense the wet laundry amount of the laundry soaked with the wash water after completion of supply of the wash water, through rotation of the drum.

In this case, the wet laundry amount may be sensed by detecting an amount of current consumed by the drive motor 121 while rotating the drum 130, in which laundry is loaded, at a predetermined speed for a predetermined time by the drive motor 121, and detecting an RPM of the drum 130 through the Hall sensor (not shown) equipped in the drive motor 121 when the drum 130 rotates by inertia in accordance with the weight of the laundry after cutting off power supplied to the drive motor 121. Meanwhile, the wet laundry amount of the laundry loaded in the drum 130 may be calculated by comparing the detected current amount of the drive motor 121 and the detected inertial RPM of the drum 130 with the wet laundry table (not shown).

Meanwhile, the wet laundry spin-drying step S114 is configured to spin-dry the laundry soaked with the wash water at a high speed and to sense a degree of unbalance of the spin-dried laundry. In the wet laundry spin-drying step S114, the drum 130 may be rotated at a higher speed than in the wet laundry amount sensing step S113. For example, the wet laundry spin-drying step S114 may perform spin-drying by accelerating the drum 130 to rotate in one direction at 90 to 110 RPM.

The wet laundry spin-drying step S114 as described above is configured to determine a wet laundry amount and a laundry quality based on a degree of unbalance of the laundry spin-dried in the wet laundry spin-drying step S114. The wet laundry spin-drying step S114 may read the degree of unbalance of the laundry by accelerating the rotation speed of the drum in multiple stages up to a predetermined speed. In a wet laundry unbalance sensing step S337, the rotation speed of the drum 130 may be accelerated from 90 to 110 RPM to 700 to 800 RPM to sense unbalance of the laundry.

In this case, the rotation direction of the drum 130 may be clockwise or counterclockwise, and the drum 130 may repeatedly rotate at least one or more, for sensing of a degree of unbalance.

In this case, in the middle of acceleration of the drum 130 from 90 to 110 RPM to 700 to 800 RPM, rotation of the drum 130 may be maintained at a predetermined speed for a predetermined time to stabilize the laundry. The predetermined speed of the drum 130 may be maintained at 400 to 500 RPM.

The humid laundry amount sensing step S115 is configured to calculate a moisture content of the laundry spin-dried in the wet laundry spin-drying step S114. The humid laundry amount sensing step S115 may calculate a humid laundry amount by detecting an amount of current required for driving of the drum 130 and an inertial RPM of the drum 130, and comparing the detected current amount of the drive motor 121 and the detected inertial RPM of the drum 130 with the wet laundry table (not shown), similarly to the dry laundry amount sensing step S111.

The laundry amount/quality calculation step S116 calculates an accurate laundry amount and an accurate laundry quality through a laundry amount/quality table (not shown) using the dry laundry amount calculated in the dry laundry amount sensing step S111, the wet laundry amount calculated in the wet laundry amount sensing step S113, the degree of unbalance calculated in the wet laundry spin-drying step S114, and the humid laundry amount calculated in the humid laundry amount sensing step S115.

In this case, the laundry amount/quality table may store the laundry amount and quality of the laundry loaded in the drum 130 to use the laundry amount and quality as data of machine learning pre-trained by an artificial neural network that has been trained to classify a laundry amount and a laundry quality into certain levels in accordance with specific criteria based on dry laundry amount, water content, moisture content, and unbalance degree.

Accordingly, the laundry amount/quality calculation step S116 may determine a laundry quality in the sterilization washing procedure (for example, the first laundry quality or the second laundry quality) based on dry laundry amount, water content, moisture content, and unbalance degree, and may determine a sterilization temperature of the sterilization process S130 or S240 in accordance with the laundry quality determined in the sterilization washing procedure.

In the above-described sterilization washing procedure according to the embodiment of the present disclosure, a sterilization temperature according to the laundry quality in the sterilization process may be set based on the laundry quality determined in the laundry amount and quality sensing process S110 or S210. Accordingly, it may be possible to prevent damage to the laundry caused by steam supplied in the sterilization washing procedure.

As discussed above, the preferred embodiments of the present disclosure have been described in detail. However, a person skilled in the relevant technical field would be able to modify and implement the present disclosure in various ways without departing from the spirit and scope of the present disclosure defined in the appended claims. Therefore, any modifications to future embodiments of the present disclosure will not deviate from the scope of the present disclosure.

## Claims

1. A control method of a washing machine equipped with a tub configured to store wash water, a drum provided in the tub to be rotatable and configured to load laundry therein, and a steam generator configured to supply steam to an interior of the tub, the washing machine comprising a sterilization washing procedure of performing sterilization of the laundry, the control method comprising:
laundry amount and quality sensing of determining a laundry amount and a laundry quality of the laundry;
washing of performing a washing process in accordance with the laundry amount;
sterilization of varying a supply amount and a supply time of the steam in accordance with the laundry quality; and
rinsing of performing a rinsing process in accordance with the laundry amount.

2. The control method according to claim 1, wherein the washing further comprises:
water supply of supplying wash water to the laundry, drainage of draining the wash water used in washing after washing of the laundry; and
washing-spin-drying of spin-drying the laundry to expel the wash water from the laundry,
wherein the sterilization is performed after the washing-spin-drying.

3. The control method according to claim 1, wherein the sterilization sets a sterilization temperature of the sterilization in accordance with the laundry quality sensed in the laundry amount and quality sensing.

4. The control method according to claim 3, wherein the sterilization temperature is set in accordance with the supply amount and the supply time of the steam.

5. The control method according to claim 3, wherein the tub is further equipped with a temperature sensor configured to sense a temperature inside the tub, and the sterilization temperature is sensed by the temperature sensor.

6. The control method according to claim 1, wherein the laundry amount and quality sensing comprises:
dry laundry amount sensing of sensing a dry laundry amount of the laundry loaded in the drum;
laundry amount sensing-water supply of supplying a predetermined amount of wash water to the drum;
wet laundry amount sensing of sensing a wet laundry amount of the laundry soaked with the wash water;
wet laundry spin-drying of spin-drying the laundry soaked with the wash water to have the wet laundry amount;
humid laundry amount sensing of sensing a humid laundry amount of the spin-dried laundry; and
laundry amount/quality sensing of a laundry amount and a laundry quality based on the dry laundry amount, the wet laundry amount, and the humid laundry amount.

7. The control method according to claim 6, wherein the laundry amount sensing-water supply simultaneously supplies the wash water and rotates the drum at a predetermined speed.

8. The control method according to claim 6, wherein the wet laundry spin-drying further comprises wet laundry unbalance sensing of sensing unbalance of the laundry simultaneously with the spin-drying of the laundry.

9. The control method according to claim 6, wherein each of the dry laundry amount sensing, the wet laundry amount sensing, and the humid laundry amount sensing performs the sensing thereof based on a current amount of a drive motor for rotation of the drum at a predetermined speed and an RPM of the drum during inertial rotation of the drum.

10. The control method according to claim 1, further comprising preliminary rinsing of simultaneously performing rinsing and spin-drying of the laundry used in the washing before the sterilization.

11. A control method of a washing machine equipped with a tub configured to store wash water, a drum provided in the tub to be rotatable and configured to load laundry therein, and a steam generator configured to supply steam to an interior of the tub, the washing machine comprising a sterilization washing procedure of performing sterilization of the laundry, the control method comprising:
laundry amount and quality sensing of determining a laundry amount and a laundry quality of the laundry;
washing of performing a washing process in accordance with the laundry amount;
first rinsing of performing a first rinsing process and rinsing-spin-drying in accordance with the laundry amount;
sterilization of varying a supply amount and a supply time of the steam in accordance with the laundry quality; and
second rinsing of performing a second rinsing process in accordance with the laundry amount.

12. The control method according to claim 11, wherein the sterilization sets a sterilization temperature of the sterilization in accordance with the laundry quality sensed in the laundry amount and quality sensing.

13. The control method according to claim 12, wherein the sterilization temperature is set in accordance with the supply amount and the supply time of the steam.

14. The control method according to claim 12, wherein the tub is further equipped with a temperature sensor configured to sense a temperature inside the tub, and the sterilization temperature is sensed by the temperature sensor.

15. The control method according to claim 11, wherein the laundry amount and quality sensing comprises:
dry laundry amount sensing of sensing a dry laundry amount of the laundry loaded in the drum;
laundry amount sensing-water supply of supplying a predetermined amount of wash water to the drum;
wet laundry amount sensing of sensing a wet laundry amount of the laundry soaked with the wash water;
wet laundry spin-drying of spin-drying the laundry soaked with the wash water to have the wet laundry amount;
humid laundry amount sensing of sensing a humid laundry amount of the spin-dried laundry; and
laundry amount/quality sensing of a laundry amount and a laundry quality based on the dry laundry amount, the wet laundry amount, and the humid laundry amount.

16. The control method according to claim 15, wherein the laundry amount sensing-water supply simultaneously supplies the wash water and rotates the drum at a predetermined speed.

17. The control method according to claim 15, wherein the wet laundry spin-drying further comprises wet laundry unbalance sensing of sensing unbalance of the laundry simultaneously with the spin-drying of the laundry.

18. The control method according to claim 15, wherein each of the dry laundry amount sensing, the wet laundry amount sensing and the humid laundry amount sensing performs the sensing thereof based on a current amount of a drive motor for rotation of the drum at a predetermined speed and an RPM of the drum during inertial rotation of the drum.
